# EUROPÄISCHE PATENTSCHRIFT

(11) **EP 4 479 126 B1**
(45) Veröffentlichungstag und Bekanntmachung des Hinweises auf die Patenterteilung: **23.09.2026**
(21) Anmeldenummer: 23705257.6
(22) Anmeldetag: 14.02.2023
(51) Int. Cl.: A61N 1/04, A61N 1/36

(54) **VORRICHTUNG UND VERFAHREN ZUR ELEKTRISCHEN STIMULATION VON KÖRPERBEREICHEN**
DEVICE AND METHOD FOR THE ELECTRICAL STIMULATION OF BODY REGIONS
DISPOSITIF ET PROCÉDÉ DE STIMULATION ÉLECTRIQUE DE RÉGIONS CORPORELLES

(30) Priorität: 16.02.2022 DE 102022103581; 16.02.2022 DE 202022100862 U; 25.03.2022 DE 102022107069
(43) Veröffentlichungstag der Anmeldung: 25.12.2024
(73) Patentinhaber: Eaglefit GmbH, 89129 Ulm/Langenau (DE)
(72) Erfinder: BORNER, Jochen, 89129 Ulm/Langenau (DE)
(74) Vertreter: Raunecker, Klaus Peter
(86) Internationale Anmeldenummer: PCT/EP2023/053649
(87) Internationale Veröffentlichungsnummer: WO 2023/156391

(56) Entgegenhaltungen:
- DE-A1- 102018 101 544
- DE-U1- 202020 106 704
- US-A1- 2010 185 259
- US-A1- 2015 032 184

## Beschreibung

Die Erfindung betrifft eine Vorrichtung zur Stimulation von Körperbereichen eines Benutzers nach dem Oberbegriff des Anspruchs 1, wie sie beispielsweise aus DE 20 2020 106 704 U1 und DE 10 2018 101544 als bekannt hervorgeht. Weiterhin betrifft die Erfindung ein Verfahren zur Stimulation von Körperbereichen.

Solche Stimulationsvorrichtungen werden beispielsweise zum elektrischen Muskelstimulationstraining (EMS) ausgewählter Körperbereiche eines Benutzers im Rahmen von Rehabilitationsmaßnahmen oder zum sportlichen Training verwendet. Die Vorrichtung umfasst ein Bekleidungsstück, das zumindest abschnittsweise aus einem elastischen Textilgebilde besteht und am Körper der trainierenden Person getragen wird. In dem Bekleidungsstück ist mindestens ein Paar von Elektroden angeordnet, die die gewünschte elektrische Muskelstimulation bewirken, wobei diese Elektroden mittels elektrischer Leitungen an eine Spannungs- oder Stromquelle angebunden ist, mittels derer die Elektroden mit elektrischer Energie versorgt werden. Weiterhin umfasst die Vorrichtung mindestens eine Steuereinheit, mit deren Hilfe die beispielsweise einstellbar pulsierende Spannungsversorgung der Elektroden gesteuert wird.

Aus der DE 20 2020 106 704 U1 ist ein Bekleidungsstück aus einem textilen Material bekannt, in dem mindestens ein Elektrodenpaar integriert sind. Während der Benutzung des Bekleidungsstücks kontaktieren die Elektroden wenigstens einen Körperbereich der trainierenden Person. Die Spannungsversorgung der Elektroden wird während des Trainings mittels einer Steuereinheit angesteuert, die an dem Bekleidungsstück befestigt werden kann. Zur Anbindung der Steuereinheit an das Bekleidungsstück ist das Bekleidungsstück mit einer Trägereinheit versehen, die mit den Elektroden elektrisch verbunden ist. An dieser Trägereinheit kann die Steuereinheit mit Hilfe von Fixierelementen eingerastet und befestigt werden.

Der Erfindung liegt die Aufgabe zugrunde, eine verbesserte Vorrichtung zum elektrischen Muskelstimulationstraining mit einem Bekleidungsstück zur Stimulation von Körperbereichen eines Benutzers bereitzustellen. Weiterhin soll ein Verfahren zur elektrischen Stimulation von Körperbereichen vorgeschlagen werden.

Diese Aufgabe wird gelöst durch eine Vorrichtung mit den Merkmalen des Anspruchs 1 und ein Verfahren mit den Merkmalen des Anspruchs 9.

Die Unteransprüche betreffen vorteilhafte Weiterbildungen und Varianten der Erfindung.

Die Vorrichtung zur Stimulation von Körperbereichen eines Benutzers umfasst ein Bekleidungsstück aus einem elastischen Textilgebilde mit integrierten Elektroden sowie eine lokale Steuereinheit, die der Steuerung und/oder Energieversorgung der Elektroden dient. In diese Steuereinheit ist - neben weiteren Komponenten - eine Spannungsquelle, beispielsweise ein Lithium-Ionen-Akku, zur Energieversorgung der Elektroden integriert. Für einen störungsfreien Betrieb der Vorrichtung muss während des Trainings eine kontinuierliche Strom- bzw. Spannungsversorgung und Steuerung der Elektroden des Bekleidungsstücks gewährleistet sein. Die Steuereinheit muss daher in einer solchen Weise sicher und fest mit dem Bekleidungsstück verbunden sein, dass auch bei starken und ruckartigen Bewegungen des Benutzers ein stabiler Kontakt zwischen den Elektroden des Bekleidungsstücks und der Steuereinheit gegeben ist.

Insbesondere kann das Bekleidungsstück erfindungsgemäß eine insbesondere als verschließbare Aufnahmevorrichtung ausgebildete Tasche aufweisen, in der die Steuereinheit während der Benutzung des Bekleidungsstücks aufgenommen ist. In einem Innenbereich der Aufnahmevorrichtung bzw. der Tasche befindet sich eine Kontaktiereinheit, an der die Elektroden über Zuleitungen elektrisch angebunden sind und an die die Steuereinheit elektrisch angekoppelt werden kann. Da die Steuereinheit in einer Tasche bzw. einer verschließbaren Aufnahmevorrichtung des Bekleidungsstücks aufgenommen ist, wird sichergestellt, dass auch bei bewegungsintensiven Aktivitäten wie dem Springen auf einem Trampolin, dem Joggen oder auch komplexen Yoga- oder HIIT-Übungen eine sichere Anbindung der Spannungsversorgung der Steuereinheit an die Zuleitungen der Elektroden aufrechterhalten bleibt.

Die Aufnahmevorrichtung bzw. die Tasche befindet sich vorteilhafterweise im Brust- oder Hüftbereich des Bekleidungsstücks, also in einem Bereich, in dem sie die Bewegungsfreiheit des Benutzers nicht beeinträchtigt.

In einer Variante der Erfindung ist die Steuereinheit fest, d.h. unlösbar mit der Kontaktiereinheit verbunden. Dabei kann die Aufnahmevorrichtung bzw. die Tasche vorteilhafterweise als allseitig geschlossener Hohlraum in dem Bekleidungsstück ausgebildet sein, so dass die Steuereinheit unlösbar und von außen her unzugänglich in dem Bekleidungsstück integriert ist. Die Steuereinheit ist vorteilhafterweise gekapselt, beispielsweise mit einer Kunststoffhaut versehen, um sie gegenüber einem Eindringen von Schweiß, Wasser etc. zu schützen und eine Reinigung des Bekleidungsstücks, beispielsweise ein Waschen in der Waschmaschine, zu ermöglichen, ohne die Steuereinheit zu beschädigen. Die Aufladung der in der Steuereinheit integrierten Spannungsquelle erfolgt in diesem Fall vorteilhafterweise induktiv vor bzw. nach der Benutzung des Bekleidungsstücks, beispielsweise mit Hilfe einer induktiven Ladestation, auf die das Bekleidungsstück aufgelegt bzw. aufgespannt wird.

Alternativ zu einer solchen umseitig geschlossenen Ausführungsform kann die Aufnahmevorrichtung auch eine in das Bekleidungsstück eingewebte, aufgenähte oder in ähnlicher Weise befestigte Tasche mit einer (vorteilhafterweise verschließbaren) Öffnung sein. Die Öffnung ermöglicht eine Zugänglichkeit der Steuereinheit von außen, so dass einzelne Komponenten der Steuereinheit, insbesondere ein als Spannungsquelle dienender und in die Steuereinheit eingesteckter Akku, entnommen werden kann. Auf diese Weise kann der Akku vor bzw. nach der Benutzung des Bekleidungsstücks außerhalb des Bekleidungsstücks induktiv oder durch Einstecken in eine Ladeeinheit aufgeladen werden.

In einer weiteren Ausgestaltung der Erfindung ist die Steuereinheit lösbar mit der Kontaktiereinheit oder einer Steckverbindung verbunden. Die Tasche des Bekleidungsstücks weist eine Öffnung auf, durch die die Steuereinheit aus dem Bekleidungsstück herausgenommen werden kann, beispielsweise um die in der Steuereinheit integrierte Spannungsquelle aufzuladen oder zu ersetzen, um die Steuereinheit in ein anderes Bekleidungsstück einzusetzen, um Wartungen an der Steuereinheit durchzuführen etc. Um die Lage der Steuereinheit während des Trainings in der Tasche des Bekleidungsstücks zu sichern, ist die Tasche vorteilhafterweise mit einem Verschlusssystem, beispielsweise einem Reißverschluss, einem Klettverschluss, einem Druckknopf etc. versehen.

Die elektrische Anbindung der Steuereinheit an die Kontaktiereinheit im Innenraum der Tasche erfolgt vorteilhafterweise mittels einer Steckverbindung und/oder mit Hilfe einer magnetischen Positionierung der korrespondierenden Kontaktelemente auf Steuereinheit und Kontaktiereinheit.

Alternativ zur oben beschriebenen Ausführungsform in Form einer (allseits geschlossenen oder verschließbaren) Tasche kann die Aufnahmevorrichtung als ein fest mit dem Bekleidungsstück verbundener, wasserdicht verschließbarer Behälter ausgestaltet sein, in den die Steuereinheit eingelegt ist bzw. eingelegt werden kann. Der Behälter kann insbesondere aus zwei durch Scharniere verbundenen Kunststoffschalen bestehen, wobei zwischen den Kunststoffschalen eine umlaufende Dichtung vorgesehen ist, die im geschlossenen Zustand des Behälters ein Eindringen von Wasser, Schweiß etc. in den Innenraum des Behälters verhindert. Im Innenraum des Behälters ist die Kontaktiereinheit angeordnet, die fest oder lösbar mit der Steuereinheit verbunden sein kann.

Zusätzlich zu der Spannungsquelle ist in die Steuereinheit vorteilhafterweise ein Modul zur Datenübertragung integriert, mit dem die Steuereinheit von einer Basisstation, beispielsweise einem Handy, angesteuert werden kann. Die Datenübertragung kann zweckmäßigerweise einerseits über Bluetooth (für Anwendungen im häuslichen Umfeld), andererseits aber auch über Zigbee (für Anwendungen in einem Studioumfeld) oder/oder über einen anderen Standard erfolgen, wobei die Steuereinheit von sich aus den dem jeweiligen Umfeld angepassten Datenübertragungsmodus erkennt und entsprechend wählt. - Alternativ zur Integration in die Steuereinheit kann das Modul zur Datenübertragung auch direkt in das Bekleidungsstück integriert sein.

In einer vorteilhaften Variante der Erfindung kann die Vorrichtung weiterhin eine visuelle und/oder akustische Stimulationseinheit umfassen, welche dazu eingerichtet ist, einem Benutzer visuelle oder akustische Reize auf Basis von seitens der Basisstation übertragenen Signalen zur Verfügung zu stellen. Wie bereits erwähnt kann es sich bei der Basisstation insbesondere um ein mobiles Endgerät, wie ein Mobiltelefon oder ein Tablet in Verbindung mit einer passenden App handeln.

Dabei kann die Basisstation dazu eingerichtet sein, Signale und Frequenzen zur Erzeugung von Reizen zu übermitteln, welche die Intensität von EEG-Frequenzbändern beeinflussen, also beispielsweise im Delta-, Theta-, Beta-, oder Gamma-Frequenz-Bereich. Auf diese Weise könnten mittels einer Anwendung auf einem mobilen Endgerät zusätzlich zu den Elektroden auf einem Kleidungsstück Kopfhörer und/oder Lichtquellen, beispielsweise LEDs in einer Brille derart rhythmisch angesprochen werden, dass sich in einem EEG das entsprechende gewünschte Frequenzmuster einstellt.

Wenn die Basisstation dazu eingerichtet ist, Signale zur Erzeugung akustischer und oder optischer Reize zu übermitteln, welche auf die Ansteuerung der Elektroden abgestimmt sind, können sich in Verbindung mit den gleichzeitig angesprochenen Elektroden des Kleidungsstücks die Trainingsmotivation und -effektivität weiter steigern lassen.

Die beschriebene Variante eignet sich insbesondere dazu, das Gehirn in einen bestimmten Zustand zu bringen, um z.B. Lerninhalte mittels Suggestion zu vermitteln oder Ähnliches zu erreichen.

Mit anderen Worten kann die Vorrichtung dazu eingerichtet sein, periodische Stimulationen eines Benutzers über mehrere unterschiedliche Wege gleichzeitig zu erzeugen, insbesondere über
- Akustische Stimulation, beispielsweise mittels eines Kopfhörers
- Optische Stimulation, beispielsweise mittels einer mit Lichtquellen, insbesondere LED's, ausgestatteten Brille
- Elektrische Stimulation, beispielsweise über die Elektroden eines EMS-Anzuges
- Taktile Stimulation, beispielsweise über eine entsprechende Aktuatorik, welche beispielsweise in einen EMS-Anzug integriert sein kann
- Magnetische Stimulation, beispielsweise über durch eine Magnetfeldmatte erzeugte, insbesondere periodische Magnetfelder

Dadurch, dass die Frequenzen der jeweiligen Stimulationen auf einander abgestimmt, insbesondere ähnlich oder identisch sind bzw. ganzzahlige Vielfache voneinander sind und hinsichtlich ihrer Phasenlage an einander angeglichen sind, so dass eine Form von Gleichtakt entsteht, kann eine erhöhte Effektivität beim Erzeugen eines gewünschten Zustandes erreicht werden. Beispielsweise kann das Gehirn des Nutzers in einen speziellen Frequenzbereich versetzt werden, durch welchen ein erhöhtes Potenzial für Superlearning oder andere Methoden kognitiver Konditionierung erreicht werden kann.

Es versteht sich von selbst, dass auch neben einer Kombination aller oben genannten Möglichkeiten auch Unterkombinationen der Möglichkeiten sinnvoll zur Anwendung kommen können.

Soll das Bekleidungsstück zur Ausstattung eines Fitness-Studios gehören, in dem mehrere Personen gleichzeitig trainieren, so kann zusätzlich im Brust-, Schulter- oder Hüftbereich des Bekleidungsstücks ein Identifikationsmodul, beispielsweise ein NFC-Chip, befestigt sein. Dieses Identifikationsmodul dient dazu, das Bekleidungsstück (bzw. seinen momentanen Benutzer) in einer Basisstation des Studios einem ausgewählten Trainingsprogramm zuordnen zu können.

Weiterhin können in das Bekleidungsstück Sensoren integriert sein, mit deren Hilfe trainingsbegleitend Messwerte des physischen Zustands des Benutzers (z.B. Blutdruck, Puls, Laktatwert etc.) erfasst werden. Vorteilhafterweise werden die Messwerte der Sensoren online in der Steuereinheit ausgewertet und/oder über das in der Steuereinheit integrierte Datenübertragungsmodul an die Basisstation zur Auswertung übertragen. Dies ermöglicht ein trainingsbegleitendes Monitoring medizinischer Daten des Benutzers und erlaubt ein simultanes Eingreifen, z.B. im Fall einer Überbeanspruchung des Benutzers, sowie eine Kontrolle dahingehend, ob bestimmte Trainings- bzw. Behandlungsabläufe korrekt ausgeführt werden. Weiterhin kann das Training auf den körperlichen Zustand des Benutzers hin optimiert werden, indem z.B. ausgewählte Elektroden in einem solchen Modus betrieben werden, der in möglichst geringer Zeit oder einem möglichst geringen körperlichen Aufwand zu einem optimalen Trainingsergebnis führt.

In das Bekleidungsstück sind Aktoren integriert, mit denen taktile Reize (z.B. Berührung, Druck, Temperatur ...) an die Haut des Benutzer übertragen werden können. Auf diese Weise kann das Bekleidungsstück als Teil einer virtuellen Umgebung zum Einsatz kommen, in der ein Benutzer in einem virtuellen Umfeld unterschiedliche Sportarten durchführen, in einem virtuellen Arbeitsumfeld trainiert oder realitätsnah therapiert werden kann.

Nachfolgend werden Ausführungsbeispiele und Varianten der Erfindung anhand der Zeichnung näher erläutert. Es zeigen:
- Fig. 1: eine schematische Vorderansicht eines Bekleidungsstücks zum elektrischen Muskelstimulationstraining (EMS) ausgewählter Körperbereiche eines Benutzers;
- Fig. 2a: eine schematische Detailansicht einer in einer Tasche des Bekleidungsstücks aufgenommenen Steuereinheit gemäß dem Bereich II - II in Figur 1;
- Fig. 2b:: eine schematische Schnittansicht der Tasche der Fig. 2a gemäß der Schnittlinie IIb **-** IIb in Figur 2a;
- Fig. 3:: eine schematische Schnittansicht einer alternativen Ausgestaltung der Tasche;
- Fig. 4:: eine schematische Detailansicht einer Steuereinheit, die in einem mit dem Bekleidungsstück verbundenen Behälter aufgenommen ist.

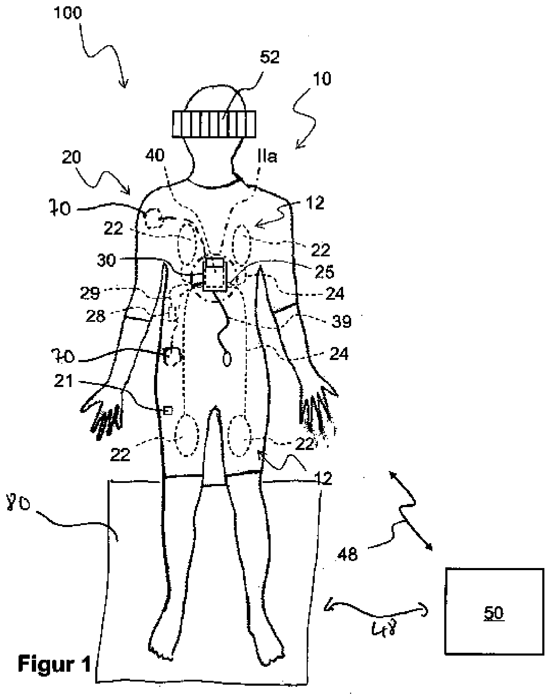

Figur 1 zeigt in einer schematischen Darstellung einen Benutzer 10 bei der Nutzung einer erfindungsgemäßen Vorrichtung 100 zur elektrischen Muskelstimulation ausgewählter Körperbereiche, wozu der Benutzer 10 ein Bekleidungsstück 20 in Form eines Trikots mit kurzen Ärmeln und kurzen Beinen trägt. Das Bekleidungsstück 20 besteht zumindest abschnittsweise aus einem elastischen textilen Material und enthält auf einer dem Benutzer 10 zugewandten Innenseite mehrere Elektrodenpaare 22, die in ausgewählten Körperbereichen 12 des Benutzers 10 angeordnet sind und deren Außenkonturen in Figur 1 durch gestrichelte Linien angedeutet sind. Die Elektrodenpaare 22 sind in einer solchen Weise an der Innenseite des Bekleidungsstücks 20 angeordnet, dass sie während der Benutzung die Haut des Benutzers 10 in den zu stimulierenden Körperbereichen 12 kontaktieren. Die Elektrodenpaare 22 können beispielsweise durch flexible leitfähige Folien gebildet sein, die an der Innenseite des Bekleidungsstücks 20 befestigt sind. Alternativ können sie durch ein leitfähiges elastisches Garn gebildet sein, welches lokal in das (elektrisch nichtleitende) elastische Material des Bekleidungsstücks 20 eingearbeitet, beispielsweise eingestrickt oder eingestickt ist. Im Ausführungsbeispiel der Figur 1 sind Elektrodenpaare 22 im Bereich des rechten und des linken Oberschenkels des Benutzers 10 sowie ein Elektrodenpaar 22 im Brustbereich des Benutzers 10 integriert. In das Bekleidungsstück 20 sind weiterhin Sensoren 28 eingearbeitet, mit denen trainingsrelevante Messgrößen bzw. Bewegungsdaten des Benutzers erfasst werden, beispielsweise BIA-Sensoren, Feuchtigkeitssensoren, Blutzucker-Sensoren, Laktatsensoren, Berührungssensoren, Bewegungssensoren, Magnetwiderstands-Sensoren, etc. Einer dieser Sensoren 28 ist in Figur 1 schematisch und gestrichelt dargestellt. Zur Kontaktierung der Elektroden 22 sowie der Sensoren 28 sind elektrischer Zuleitungen 24, 29 vorgesehen, die in das Textil des Bekleidungsstücks 20 integriert sind, und die mit einer ebenfalls in das Textil des Bekleidungsstücks integrierten Kontaktiereinheit 25, beispielsweise einem Flachbandkabel, verbunden sind.

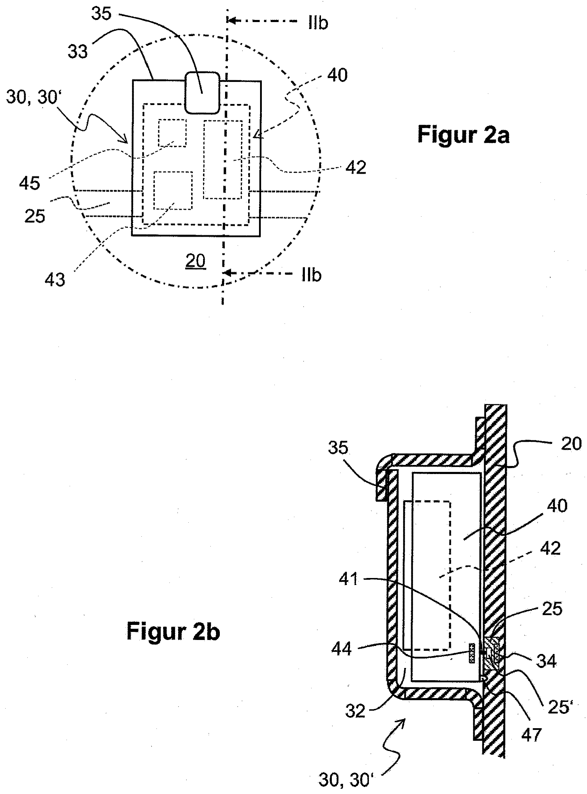

Zur Ansteuerung und Spannungsversorgung der Elektroden 22 sowie zur Spannungsversorgung und Messwerterfassung der Sensoren 28 ist eine Steuereinheit 40 vorgesehen, die eine Spannungsquelle in Form einer wiederaufladbaren Batterie 42, beispielsweise eines Lithium-Ionen-Akkus, enthält, durch den die Elektroden 22 und Sensoren 28 mit Energie versorgt werden (siehe die Detailansicht der Figur 2a). Weiterhin enthält die Steuereinheit 40 einen Mikrocontroller 43, mit dem vorgegebene Anregungszyklen der Elektroden 22 gesteuert und Messwerte der Sensoren 28 erfasst und gespeichert werden können. Außerdem enthält die Steuereinheit 40 ein Modul 45 zum Datenaustausch mit einer Basisstation 50, beispielsweise ein Bluetooth-Modul oder ein Zigbee-Modul. Damit kann beispielsweise ein bestimmtes Trainingsprogramm berührungslos an die Steuereinheit 40 übertragen werden, oder Benutzerdaten, die während des Trainings mittels der in dem Bekleidungsstück 20 integrierten Sensoren 28 gewonnen werden, können berührungslos an die Basisstation 50 transferiert werden. Ein solcher Datentransfer bzw. Datenaustausch ist in Figur 1 symbolisch durch einen Doppelpfeil 48 gekennzeichnet.

Während der Benutzung des Bekleidungsstücks 20 muss die Steuereinheit 40 ununterbrochen elektrisch mit den Elektroden 22 verbunden sein, um die gewünschte Ansteuerung und Spannungsversorgung der Elektroden 22 sicherzustellen. Hierzu ist das Bekleidungsstück 20 mit (mindestens) einer verschließbaren Aufnahmevorrichtung 30 versehen, in der die Steuereinheit 40 aufgenommen wird und in deren Innenraum 32 die Steuereinheit 40 an die Kontaktiereinheit 25 angeschlossen werden. Diese Verbindung kann insbesondere mittels einer Steckverbindung oder einer Rastverbindung realisiert werden.

Weiterhin gezeigt in der Figur 1 ist eine Magnetfeldmatte 80, mittels welcher elektromagnetische Stimulationen für den Benutzer 10 erzeugt werden können. Der Datenaustausch mit der Magnetfeldmatte kann dabei drahtlos durch die Steuereinheit 40 erfolgen, es ist alternativ oder zusätzlich ebenso denkbar, die Magnetmatte 80 direkt von der Basisstation 50 aus anzusteuern, was über den Pfeil 48 angedeutet ist.

In einer in den Figuren 2a, 2b gezeigten Ausführungsform ist die Aufnahmevorrichtung 30 eine in das Textil des Bekleidungsstücks 20 eingearbeiteten oder aufgesetzten Tasche 30'. Die Steuereinheit 40 ist mit einer Mehrzahl stiftförmiger elektrischer Kontaktelemente 41 versehen, die aus der Steuereinheit 40 herausragen und die beim Einstecken der Steuereinheit 40 in die Tasche 30' in Kontakt mit der im Innenbereich 31 der Tasche 30' angeordneten Kontaktiereinheit 25 kommen. Um eine elektrische Verbindung zu erreichen, ist die Kontaktiereinheit 25 in einer solchen Weise in das Bekleidungsstück 20 eingearbeitet, dass Kontaktfelder 25' der Kontaktiereinheit 25, in die die Zuleitungen 24, 29 münden (und die also die in elektrischer Verbindung mit den Elektroden 22 und den Sensoren 28 stehen) im Innenbereich der Tasche 30' den Kontaktelementen 41 der Steuereinheit 40 gegenüberliegen, so dass die Kontaktelemente 41 der Steuereinheit 40 an diese Kontaktfelder 25' der Kontaktiereinheit 25 angeschlossen werden können. Dabei ist es wichtig, die korrekte Paarung zugehöriger Kontaktelemente 41 und Kontaktfelder 25' zu gewährleisten. Der Anschluss kann beispielsweise mittels einer Flachbandkabel-Steckverbindung oder einem anderen Stecksystem realisiert werden, insbesondere kann die Steuereinheit 40 in einen in der Tasche 30' vorgesehenen (in den Figuren aber nicht gezeigten) Aufnahmeschuh eingesteckt werden, der über eine mechanische Fixierung der Steuereinheit 40 eine korrekte Verbindung der Kontaktelemente 41 des Steuereinheit 40 mit den Kontaktfeldern 25' der Kontaktiereinheit 25 gewährleistet. Alternativ kann - wie in Figur 2b gezeigt - sowohl die Kontaktiereinheit 25 als auch die Steuereinheit 40 mit Magneten 34, 44 versehen sein, die eine korrekte Positionierung der einzelnen Kontaktelemente 41 der Steuereinheit 40 gegenüber den zugehörigen Kontaktfeldern 25' der Kontaktiereinheit 25 sicherstellen. Zusätzlich können z.B. Rastnasen 47 vorgesehen sein, die eine mechanische Positionierung der Steuereinheit 40 gegenüber der Kontaktiereinheit 25 gestatten.

Um die Steuereinheit 40 verliersicher in der Tasche 30' aufzunehmen, ist die Tasche 30' vorteilhafterweise mit einem Verschlusssystem 35, beispielsweise mit einem Reißverschluss oder einem Klettband, versehen. Ein solches Verschlusssystem 35 hält die Steuereinheit 40 während der Benutzung des Bekleidungsstücks 20 in der gewünschten Lage, stellt aber gleichzeitig sicher, dass die Steuereinheit 40 schnell und unkompliziert aus der Tasche 30' des Bekleidungsstücks 20 entnommen werden kann, damit einerseits vor/nach dem Training die in der Steuereinheit 40 integrierte Batterie 42 aufgeladen werden kann und andererseits das Bekleidungsstück 20 gereinigt bzw. gewaschen werden kann, ohne die Steuereinheit 40 zu beschädigen.

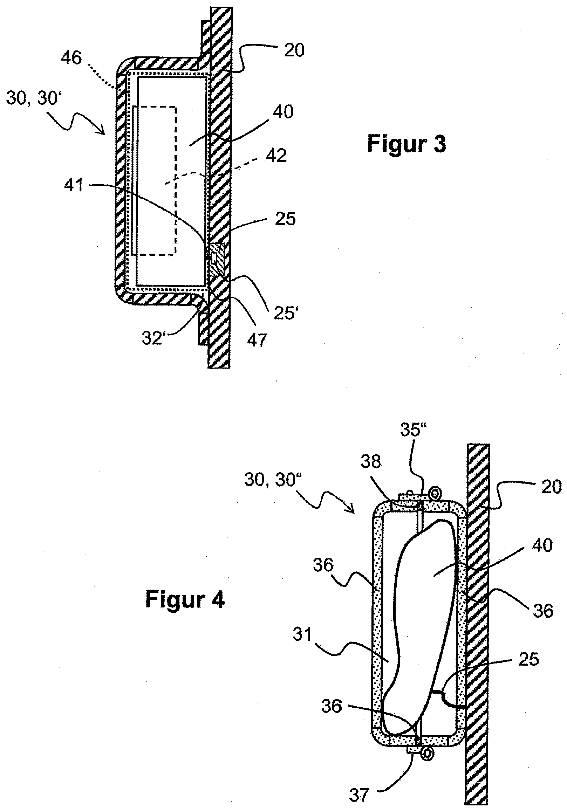

In einer in Figur 3 dargestellten alternativen Ausgestaltung ist die Steuereinheit 40 fest - also unlösbar - in das Bekleidungsstück 20 integriert, so dass die Steuereinheit 40 mitsamt des Akkus 42 ein integraler Bestandteil der Bekleidungsstücks 20 ist. Die Tasche 30' bildet dann einen geschlossenen Hohlraum 32, in den die Steuereinheit 40 aufgenommen ist. Zum Schutz der Steuereinheit 40 (inkl. des Akkus 42) vor Beeinträchtigungen durch Wasser, Schweiß etc. ist die Steuereinheit 40 von einer wasserdichten Hülle 46 umgeben. Diese Schutzhülle 46 ist so gestaltet, dass die Steuereinheit 40 bei der regelmäßigen Reinigung des Bekleidungsstücks (z.B. einer Maschinenwäsche bei 30⁰ C) nicht in ihrer Funktionstüchtigkeit beeinträchtigt wird.

Da der Akku 42 in dieser Ausführungsform nicht aus dem Bekleidungsstück 20 entnommen werden kann, erfolgt die Aufladung des Akkus 42 induktiv.

In einer weiteren - in den Figuren nicht dargestellten - Ausgestaltung ist die Steuereinheit 40 zwar fest - also unlösbar - in das Bekleidungsstück 20 integriert, der in die Steuereinheit 40 verbaute Akku bzw. Batterie 42 ist aber lösbar mit der Steuereinheit 40 verbunden. Die Tasche 30' verfügt über eine Öffnung, so dass der Akku/Batterie 42 zum Aufladen bzw. Austauschen aus der Tasche 30 entnommen werden kann.

Figur 4 zeigt eine Aufnahmevorrichtung 30 in Form eines mit dem Bekleidungsstück 20 fest verbundenen, wasserdicht verschließbaren Behälters 30". Der Behälter 30" umfasst zwei Halbschalen 36 aus einem Kunststoff, die über ein Scharnier 37 miteinander verbunden sind und im geschlossenen Zustand des Behälters 30" mittels eines Verschlusses 35" relativ zueinander fixiert werden. Zwischen den Halbschalen 36 ist ein umlaufender Dichtungsring 38 vorgesehen, der ein Eindringen von Wasser, Schweiß etc. in den Innenraum 31 des Behälters 30" verhindert. Die Kontaktiereinheit 25 ist über eine Flachbandkabel-Steckverbindung mit der im Behälter 30" aufgenommenen Steuereinheit 40 verbunden. Wie in Figur 4 dargestellt, braucht die Steuereinheit 40 keineswegs die in den Figuren 2 und 3 gezeigte rechteckige Form zu haben, sondern kann eine beliebige (prismatische oder freigeformte) Gestalt haben; insbesondere kann die Steuereinheit die Form einer dreidimensionalen Figur bzw. eines dreidimensionalen Logos haben, kann benutzerindividuell gestaltet sein, kann mit einer weichen oder textilen Oberfläche versehen sein etc. Die Steuereinheit 40 kann weiterhin (in den Figuren nicht dargestellte) Funktionsleuchten aufweisen, die vorteilhafterweise ein Logo abbilden und beispielsweise grün leuchten können. Hierzu kann die Wand der Behälters 30" (bzw. der Stoff im Bereich der Tasche 30') durchscheinend oder als Mesh-Einsatz gestaltet sein.

Die Aufnahmevorrichtung 30 für die Steuereinheit 40 kann sich insbesondere mittig im Brustbereich oder seitlich im Hüftbereich des Bekleidungsstücks 20 befinden. In unmittelbarer Umgebung der Aufnahmevorrichtung 30 ist ein Not-Aus-Schalter in Form einer Reißleine 39 vorgesehen, mit dessen Hilfe der Benutzer 10 das Trainingsprogramm unterbrechen kann.

Die Steuereinheit 40 weist (in den Figuren nicht dargestellte) Funktionsleuchten auf, die vorteilhafterweise ein Logo abbilden und beispielsweise grün leuchten können. Hierzu kann der Stoff im Bereich der Tasche 30 durchscheinend oder als Mesh-Einsatz gestaltet sein.

In dem Bekleidungsstück 20 kann ein Identifikationsmodul 21, beispielsweise ein NFC-Chip, befestigt sein. Dieses Identifikationsmodul 21 dient dazu, das Bekleidungsstück 20 (bzw. seinen Benutzer) der Basisstation 50 des Studios einem ausgewählten Trainingsprogramm zuordnen zu können. Mit Hilfe dieses Identifikationsmoduls 21 kann über die Basisstation 50 ein spezifisches, auf den jeweiligen Benutzer zugeschnittenes, Trainingsprogramm an die Steuereinheit 40 des Bekleidungsstücks 20 gesendet und dort ausgeführt werden.

Zur Erhöhung des Tragekomforts kann das Bekleidungsstück 20 unter den Armen und im Schritt mit Mesh-Einsätzen versehen sein.

Mit Hilfe der in das Textil des Bekleidungsstücks 20 integrierten Sensoren 28 können während des Trainings prozessbegleitend Benutzerdaten (z.B. Pulsschlag, Blutdruck, Blutzuckerspiegel, Sauerstoffsättigung im Blut, lokale Körpertemperatur etc.) erfasst werden. Bei muskulären Verspannungen und/oder erhöhter Muskelaktivität können diese Sensoren 28 die Aktivität messen und an die Basiseinheit 50 übertragen, wo diese Messdaten ausgewertet bzw. mit Solldaten verglichen werden und evtl. Gegenmaßnahmen eingeleitet werden. Dies ermöglicht ein optimiertes Training, z.B. bei einem Marathonläufer, der seine Dauerbelastung erfassen und optimal einstellen möchte.

Unter Verwendung der Messwerte geeigneter integrierter Sensoren 28 ist es möglich, beispielsweise durch Biofeedback oder andere geeignete Techniken zu ermitteln, zu welchem Prozentsatz und in welcher Art die einzelnen Muskeln des Benutzers 20 während des Trainings aktiviert sind und welche Muskelgruppen in welcher Tiefe angesprochen werden, damit die Einstellung der Stimulation noch präziser oder auch automatisiert erfolgen kann. Beispielsweise kann eine Meldung "95% der weißen Skelettmuskulatur am Quadrizeps sind stimuliert" unterschiedlich interpretiert und ausgewertet werden, und die zugehörige Elektrodenanregung kann entsprechend angepasst werden.

Zusätzlich können mittels weiterer integrierter Sensoren 28 die Lage und/oder Bewegungen und/oder Beschleunigung einzelner Komponenten des Bekleidungsstücks erfasst werden. Dies bietet die Möglichkeit, evtl. in Verbindung mit einer virtuellen Umgebung oder einer Augmented Reality, entsprechende Informationen über die Bewegung des Benutzers und deren Intensität an die Basisstation 50 zu übermitteln, so dass eine realistische Bewegung in nahezu beliebigem realem oder virtuellem Umfeld simuliert werden kann. Hierbei kann der Benutzer 20 z.B. mit Hilfe einer (in Figur 1 angedeuteten) 3D-Brille 52 mit einer virtuellen Umgebung, beispielsweise einem virtuellen Trainer, interagieren. Dabei kann die Brille 52 auch (in der Figur nicht dargestellte) Kopf- oder Ohrhörer zur Erzeugung akustischer Signale umfassen. Mittels der Sensoren 28 können Messwerte erfasst werden, die Aufschluss über die korrekte Durchführung der auszuführenden Bewegung geben. Mit Hilfe eines solchen Feedbacks können unterschiedlichste Bewegungsabläufe erlernt und optimiert werden, beispielsweise im Training für sportliche Aktivitäten, beim Erlernen und Optimieren von Arbeitsabläufen, in Spielsituationen etc. Alternativ oder zusätzlich können das Bekleidungsstück 20 Aktoren 70 integriert sein, mit denen taktile Reize (z.B. Berührung, Druck, Temperatur ...) an die Haut des Benutzer 10 übertragen werden können, um eine noch realitätsnähere Trainingsumgebung zu simulieren. Lediglich beispielhaft sind in der Figur 2 dieser Aktoren 70 schematisch angedeutet. In einer solchen virtuellen Umgebung kann der Wunsch bestehen, besondere Beanspruchungen einzelner Muskeln durch elektrische Frequenzen zu simulieren, die über die Elektroden des Bekleidungsstücks übertragen werden. So kann z.B. ein virtuelles Skifahren auf einer Buckelpiste, Joggen, Radfahren (auch Downhill) oder ein virtuelles Fitness-Studio mit virtuellen Gewichten und Maschinen ebenso simuliert werden wie ein Fahren auf einer Achterbahn.

Die Steuereinheit 40 kann weiterhin mit einem WLAN-Chip ausgestattet sein, der es einer entfernten Person, beispielsweise einem Trainer, oder einer entfernten Hard-/Software ermöglicht, die Intensität der elektrischen Simulation einzelner Muskel aus der Ferne zu steuern und/oder Daten über den körperlichen Zustand des Benutzers 10 (z.B. Puls, Blutdruck, ...) auszulesen. Auf diese Weise kann ein Benutzer 10 des Bekleidungsstücks 20 an einem virtuellen Training, an Wettkämpfen oder Spielen teilnehmen und dabei eine möglichst realistische Wahrnehmung, Steuerung, Belastung und Bewegung erfahren.

### Bezugszeichenliste

- 100: Vorrichtung
- 10: Benutzer
- 12: Körperbereich des Benutzers
- 20: Bekleidungsstück
- 21: Identifikationsmodul
- 22: Elektroden
- 24: Zuleitungen zu den Elektroden
- 25: Kontaktiereinheit 25' Kontaktfelder
- 28: Sensor
- 29: Elektrische Leitungen zum Sensor
- 30: Aufnahmevorrichtung; 30' Tasche; 30" Behälter
- 31: Innenraum der Aufnahmevorrichtung
- 32: geschlossener Hohlraum
- 33: Öffnung der Tasche
- 34: Magnete in der Kontaktiereinheit
- 35, 35": Verschluss
- 36: Halbschale
- 37: Scharnier
- 38: Dichtung
- 39: Reißleine
- 40: Steuereinheit
- 41: Kontaktelement
- 42: Batterie / Akku
- 43: Mikrocontroller
- 44: Magnet in Steuereinheit
- 45: Bluetooth-Modul
- 46: Wasserdichte Hülle
- 47: Rastnasen
- 48: Kommunikation mit einer Basisstation
- 50: Basisstation
- 52: 3D-Brille
- 70: Aktor
- 80: Magnetfeldmatte

## Patentansprüche

1. Vorrichtung (100) zur Stimulation von Körperbereichen (12) eines Benutzers (10), wobei die Vorrichtung (100) ein Bekleidungsstück (20) aus einem elastischen Textilgebilde mit integrierten Elektroden (22) und integrierten elektrischen Zuleitungen (24) zu den Elektroden (22) sowie eine lokale Steuereinheit (40) umfasst, undwobei das Bekleidungsstück (20) eine Kontaktiereinheit (25) aufweist, die mit den Zuleitungen (24) der Elektroden (22) elektrisch verbunden ist,
**dadurch gekennzeichnet, dass**
in das Bekleidungsstück (20) mindestens ein Aktor integriert ist, mit dessen Hilfe ein taktiler Reiz auf die Haut des Benutzers (10) ausgeübt werden kann.

2. Vorrichtung nach Anspruch 1,
**dadurch gekennzeichnet, dass**
die Steuereinheit (40) fest mit der Kontaktiereinheit (25) verbunden ist.

3. Vorrichtung nach Anspruch 1,
**dadurch gekennzeichnet, dass**
die Steuereinheit (40) lösbar mit der Kontaktiereinheit (25) verbunden ist.

4. Vorrichtung nach einem der vorangehenden Ansprüche,
**dadurch gekennzeichnet, dass**
die Steuereinheit (40) ein Modul (45) zur Datenübertragung zwischen der Steuereinheit (40) und einer Basisstation (50) umfasst.

5. Vorrichtung nach einem der vorangehenden Ansprüche,
**dadurch gekennzeichnet, dass**
in das Bekleidungsstück (20) ein Identifikationsmodul (21) integriert ist.

6. Vorrichtung nach einem der vorangehenden Ansprüche,
**dadurch gekennzeichnet, dass**
in das Bekleidungsstück (20) mindestens ein Sensor (28) integriert ist.

7. Vorrichtung nach einem der vorangehenden Ansprüche,
**dadurch gekennzeichnet, dass**
die Vorrichtung dazu eingerichtet ist, periodische Stimulationen eines Benutzers über mehrere unterschiedliche Wege gleichzeitig zu erzeugen

8. Vorrichtung nach Anspruch 7,
**dadurch gekennzeichnet,**
**dass** sie dazu eingerichtet ist, periodische Stimulationen eines Benutzers durch akustische Stimulation und/oder durch optische Stimulation und/oder durch elektrische Stimulation und/oder durch magnetische Stimulation und/oder durch taktile Stimulation zu erzeugen.

9. Verfahren zur Stimulation von Körperbereichen (12) eines Benutzers (10) zum sportlichen Training mit
einer Vorrichtung nach einem der vorhergehenden Ansprüche, wobei der Benutzer (10) ein Bekleidungsstück (20) aus einem elastischen Textilgebilde mit integrierten Elektroden (22) und integrierten Sensoren (28) trägt, welche an eine Steuereinheit (40) angebunden sind,
**dadurch gekennzeichnet, dass**
mit Hilfe mindestens eines Aktors, der in das Bekleidungsstück (20) integriert ist, ein taktiler Reiz auf die Haut des Benutzers (10) ausgeübt wird.

10. Verfahren nach Anspruch 9,
**dadurch gekennzeichnet, dass**
mittels der Sensoren (28) Messwerte des Benutzers (10) erfasst und mit Hilfe der Steuereinheit (40) ausgewertet werden, und dass eine Anregung der Elektroden (22) unter Verwendung dieser Messwerte erfolgt.

11. Verfahren nach einem der Ansprüche 9 oder 10,
**dadurch gekennzeichnet, dass**
periodische Stimulationen eines Benutzers über mehrere unterschiedliche Wege gleichzeitig erzeugt werden.

12. Verfahren nach Anspruch 11,
**dadurch gekennzeichnet, dass**
periodische Stimulationen eines Benutzers durch akustische Stimulation und/oder durch optische Stimulation und/oder durch elektrische Stimulation und/oder durch magnetische Stimulation und/oder durch taktile Stimulation erzeugt werden.

## Claims

1. Device (100) for stimulating body regions (12) of a user (10), wherein the device (100) comprises an article of clothing (20) with an elastic textile structure having integrated electrodes (22) and integrated electrical supply lines (24) to the electrodes (22) and comprises a local control unit (40), and wherein the article of clothing (20) comprises a contacting unit (25) which is electrically connected to the supply lines (24) of the electrodes (22),
**characterized in that**
at least one actuator is integrated into the article of clothing (20) and can be used to apply a tactile stimulus to the skin of the user (10).

2. Device according to Claim 1,
**characterized in that**
the control unit (40) is fixedly connected to the contacting unit (25).

3. Device according to Claim 1,
**characterized in that**
the control unit (40) is detachably connected to the contacting unit (25).

4. Device according to any of the preceding claims,
**characterized in that**
the control unit (40) comprises includes a module (45) for data transfer between the control unit (40) and a base station (50).

5. Device according to any of the preceding claims,
**characterized in that**
an identification module (21) is integrated into the article of clothing (20).

6. Device according to any of the preceding claims,
**characterized in that**
at least one sensor (28) is integrated into the article of clothing (20).

7. Device according to any of the preceding claims,
**characterized in that**
the device is configured to simultaneously generate periodic stimulations of a user in several different ways.

8. Device according to Claim 7,
**characterized in that**
it is configured to generate periodic stimulations of a user by acoustic stimulation and/or by visual stimulation and/or by electrical stimulation and/or by magnetic stimulation and/or by tactile stimulation.

9. Method for stimulating body regions (12) of a user (10) for athletic training using a device according to any of the preceding claims, wherein the user (10) wears an article of clothing (20) with an elastic textile structure and having integrated electrodes (22) and integrated sensors (28) which are connected to a control unit (40),
**characterized in that**
at least one actuator which is integrated into the article of clothing (20) is used to apply a tactile stimulus to the skin of the user (10).

10. Method according to Claim 9,
**characterized in that**
measured values relating to the user (10) are captured by means of the sensors (28) and evaluated using the control unit (40), and **in that** the electrodes (22) are excited using these measured values.

11. Method according to either of Claims 9 and 10,
**characterized in that**
periodic stimulations of a user are generated simultaneously in several different ways.

12. Method according to Claim 11,
**characterized in that**
periodic stimulations of a user are generated by acoustic stimulation and/or by visual stimulation and/or by electrical stimulation and/or by magnetic stimulation and/or by tactile stimulation.

## Revendications

1. Dispositif (100) de stimulation de zones corporelles (12) d'un utilisateur (10), le dispositif (100) comprenant une pièce de vêtement (20) composée d'un produit textile élastique avec des électrodes intégrées (22) et des lignes d'arrivée (24) électriques intégrées vers les électrodes (22) ainsi qu'une unité de commande locale (40), et la pièce de vêtement (20) possédant une unité de mise en contact (25) qui est reliée électriquement aux lignes d'arrivée (24) des électrodes (22),
**caractérisé en ce que**
au moins un actionneur est intégré dans la pièce de vêtement (20), à l'aide duquel un stimulus tactile peut être exercé sur la peau de l'utilisateur (10).

2. Dispositif selon la revendication 1,
**caractérisé en ce que**
l'unité de commande (40) est reliée à demeure à l'unité de mise en contact (25).

3. Dispositif selon la revendication 1,
**caractérisé en ce que**
l'unité de commande (40) est reliée de manière amovible à l'unité de mise en contact (25).

4. Procédé selon l'une des revendications précédentes,
**caractérisé en ce que**
l'unité de commande (40) comprend un module (45) destiné à la transmission de données entre l'unité de commande (40) et une station de base (50).

5. Procédé selon l'une des revendications précédentes,
**caractérisé en ce que**
un module d'identification (21) est intégré à la pièce de vêtement (20).

6. Procédé selon l'une des revendications précédentes,
**caractérisé en ce que**
au moins un capteur (28) est intégré dans la pièce de vêtement (20).

7. Procédé selon l'une des revendications précédentes,
**caractérisé en ce que**
le dispositif est conçu pour produire des stimulations périodiques d'un utilisateur simultanément par le biais de plusieurs voies différentes.

8. Dispositif selon la revendication 7,
**caractérisé en ce**
**qu'**il est conçu pour produire des stimulations périodiques d'un utilisateur par stimulation acoustique et/ou par stimulation optique et/ou par stimulation électrique et/ou par stimulation magnétique et/ou par stimulation tactile.

9. Procédé de stimulation de zones corporelles (12) d'un utilisateur (10) à des fins d'entraînement sportif avec un dispositif selon l'une des revendications précédentes, l'utilisateur (10) portant une pièce de vêtement (20) composée d'un produit textile élastique avec des électrodes intégrées (22) et des capteurs intégrés (28), lesquels sont reliés à une unité de commande (40).
**caractérisé en ce que**
un stimulus tactile est exercé sur la peau de l'utilisateur (10) à l'aide d'au moins un actionneur qui est intégré dans la pièce de vêtement (20).

10. Procédé selon la revendication 9,
**caractérisé en ce que**
des valeurs de mesure de l'utilisateur (10) sont acquises au moyen des capteurs (28) et évaluées à l'aide de l'unité de commande (40), et **en ce qu'**une excitation des électrodes (22) est effectuée en utilisant ces valeurs de mesure.

11. Procédé selon l'une des revendications 9 ou 10,
**caractérisé en ce que**
des stimulations périodiques d'un utilisateur peuvent être produites simultanément par le biais de plusieurs voies différentes.

12. Procédé selon la revendication 11,
**caractérisé en ce que**
des stimulations périodiques d'un utilisateur sont produites par stimulation acoustique et/ou par stimulation optique et/ou par stimulation électrique et/ou par stimulation magnétique et/ou par stimulation tactile.
